# FASCICULE DE BREVET EUROPEEN

(11) **EP 0 657 211 B1**
(45) Date de publication et mention de la délivrance du brevet: **24.05.2000**
(21) Numéro de dépôt: 94203490.1
(22) Date de dépôt: 01.12.1994
(51) Int. Cl.: B01J 27/12, B01J 27/125, B01J 27/138, C07C 17/00

(54) **Système catalytique comprenant un catalyseur d'hydrogénation sur un support et procédé d'hydrodéchloration d'hydrocarbures chlorofluorés**
Hydrierungsträgerkatalysator enthaltendes Katalysatorssystem und Verfahren zur Hydrodechlorierung von Fluorchlorkohlenwasserstoffen
Catalytic system comprising a supported hydrogenation catalyst and hydrodechlorination process of chlorofluorohydrocarbons

(30) Priorité: 10.12.1993 BE 9301374
(43) Date de publication de la demande: 14.06.1995
(73) Titulaire: SOLVAY (Société Anonyme), 1050 Bruxelles (BE)
(72) Inventeur: Schoebrechts, Jean-Paul, B-1390 Grez-Doiceau (BE); Wilmet, Vincent, B-1301 Wavre (BE)
(74) Mandataire: Jacques, Philippe

(56) Documents cités:
- EP-A- 0 053 657
- EP-A- 0 343 707
- EP-A- 0 349 115
- EP-A- 0 548 743
- GB-A- 621 902
- GB-A- 2 219 796

## Description

L'invention concerne les catalyseurs d'hydrogénation.

Elle concerne plus spécialement un système catalytique contenant un catalyseur d'hydrogénation comprenant au moins un métal choisi parmi les éléments du groupe VIII du tableau périodique des éléments, sur un support comprenant au moins un composé fluoré d'aluminium.

L'invention concerne également l'utilisation de ce système catalytique dans un procédé d'hydrodéchloration, au moyen d'hydrogène, d'hydrocarbures chlorés, plus particulièrement d'hydrocarbures chlorofluorés.

L'hydrodéchloration d'hydrocarbures chlorofluorés a déjà été utilisée pour fabriquer du difluorométhane à partir de chlorodifluorométhane. Ainsi, dans la demande de brevet européen EP-A-0508660 on décrit un procédé de fabrication de difluorométhane par hydrodéchloration de chlorodifluorométhane au moyen d'hydrogène en présence d'un catalyseur de palladium supporté sur du charbon actif, de la silice ou de l'alumine. Les exemples repris dans ce document montrent que seule l'utilisation d'un support en charbon actif permet d'obtenir le difluorométhane avec une sélectivité raisonnable.

Dans le brevet US-A-5202510, l'hydrodéchloration d'hydrocarbures chlorofluorés au moyen d'hydrogène est opérée en présence d'un système catalytique comprenant un élément du groupe VIII du tableau périodique des éléments sur un support comprenant un composé fluoré de l'aluminium, tel que du fluorure d'aluminium ou de l'alumine fluorée. Ce document enseigne que ces systèmes catalytiques connus ainsi que les catalyseurs supportés sur charbon actif, lorsqu'ils sont utilisés dans des procédés d'hydrodéchloration de chlorofluoroalcanes, se désactivent très vite et qu'ils nécessitent des procédés de regénération compliqués, ce qui constitue un double inconvénient.

On a maintenant trouvé un système catalytique qui évite les inconvénients précités.

L'invention concerne dès lors un système catalytique contenant, d'une part, un catalyseur d'hydrogénation comprenant au moins un métal choisi parmi les éléments du groupe VIII du tableau périodique des éléments et, d'autre part, un support dudit catalyseur, comprenant au moins un composé fluoré de l'aluminium, ce système catalytique se caractérisant en ce que le support comprend au moins un métal alcalin et/ou alcalino-terreux.

Par composé fluoré de l'aluminium, on entend désigner tout composé qui comprend au moins de l'aluminium et du fluor.

Le support du système catalytique selon l'invention contient de préférence l'aluminium et le métal alcalin et/ou alcalino-terreux dans un rapport atomique aluminium/(métal alcalin et/ou alcalino-terreux) d'au moins 0,05. D'une manière particulièrement préférée, le rapport atomique est d'au moins 0,1. Tout particulièrement préféré est un rapport atomique d'au moins 0,2. Le support contient de préférence l'aluminium et le métal alcalin et/ou alcalino-terreux dans un rapport atomique aluminium/(métal alcalin et/ou alcalino-terreux) qui ne dépasse pas 50. D'une manière particulièrement préférée, le rapport atomique ne dépasse pas 35. Tout particulièrement préféré est un rapport atomique qui ne dépasse pas 22.

Le support du système catalytique conforme à l'invention ne présente en général qu'une faible acidité. L'acidité du support, telle que l'on peut la déterminer par adsorption d'ammoniac, est habituellement inférieure ou égale à 500 micromoles/g, de préférence inférieure ou égale à 100 micromoles/g.

La surface spécifique du support du système catalytique conforme à l'invention peut varier entre de larges limites. La surface spécifique est de préférence d'au moins 1 m²/g et d'une manière particulièrement préférée, au moins égale à 3 m²/g. En général, la surface spécifique ne dépasse pas 500 m²/g.

Le volume poreux du support peut varier entre de larges limites. Il est généralement d'au moins 0,01 cm³/g et de préférence d'au moins 0,1 cm³/g. Le volume poreux du support ne dépasse habituellement pas 1 cm³/g. De préférence, il ne dépasse pas 0,5 cm³/g.

Le catalyseur d'hydrogénation du système catalytique selon l'invention comprend au moins un métal choisi parmi les éléments du groupe VIII du tableau périodique des éléments. D'une manière préférée, le catalyseur comprend du palladium. Il peut comprendre, outre du palladium, d'autres métaux, notamment ceux choisis parmi les éléments des groupes VIII et Ib du tableau périodique des éléments.

La quantité du catalyseur d'hydrogénation sur le support est avantageusement d'au moins 0,5 %, de préférence d'au moins 2 % en poids par rapport au poids du support. Habituellement, la quantité du catalyseur d'hydrogénation sur le support ne dépasse pas 15 % en poids par rapport au poids du support. De préférence, elle ne dépasse pas 10 %.

Le système catalytique selon l'invention peut être obtenu par imprégnation du support avec une solution du catalyseur. L'imprégnation peut être réalisée par n'importe quelle méthode, telle que par exemple par la technique dite du "volume poreux" (imprégnation dite "sèche") ou par la technique du "volume excédentaire" (imprégnation dite "humide") ou encore par une combinaison de ces deux techniques. Ces techniques connues sont décrites dans le traité de Charles N. SATTERFIELD "Heterogeneous catalysis in practice", 1980, Mc.Graw-Hill Book Company, New York, notamment pages 82 et 83. Le système catalytique peut être obtenu par une seule imprégnation ou par plusieurs imprégnations successives du support.

La solution d'imprégnation peut être une solution aqueuse ou organique, acide, basique ou neutre, d'un sel inorganique ou organique du métal ou de chaque métal du catalyseur. Une solution aqueuse acide est préférée. Les sels inorganiques utilisés à ce sujet peuvent par exemple être des chlorures ou des complexes ammoniacaux. Les sels organiques peuvent notamment être des acétates ou des acétylacétonates.

Dans le cas où le catalyseur comprend plusieurs métaux différents, l'introduction de ces métaux dans le système catalytique peut être effectuée par imprégnation au moyen d'une solution contenant tous les métaux, ou par imprégnation en plusieurs fois, avec des solutions distinctes. Dans ce cas, l'ordre d'introduction peut être variable.

L'imprégnation du support se fait généralement à température ambiante.

Il peut y avoir une étape de séchage après chaque imprégnation. La ou les étapes de séchage s'effectuent habituellement sous pression réduite et à une température d'au moins 80 °C. De préférence, la température de séchage ne dépasse pas 150 °C. Le support imprégné est ensuite soumis à un traitement thermique en atmosphère réductrice. L'atmosphère réductrice est avantageusement de l'hydrogène ou un mélange d'hydrogène avec un gaz inerte. La température à laquelle est effectué le traitement thermique est de préférence d'au moins 100 °C, plus particulièrement d'au moins 150 °C. En général, la température à laquelle est effectué le traitement thermique ne dépasse pas 450 °C. De préférence, elle ne dépasse pas 300 °C. La pression à laquelle est effectué le traitement thermique est de préférence d'au moins 1 bar. D'une manière particulièrement préférée, la pression ne dépasse pas 5 bar. Le traitement thermique du support imprégné peut être effectué préalablement à l'utilisation du système catalytique dans un procédé d'hydrogénation ou en même temps que le traitement d'hydrogénation.

Dans le support du système catalytique selon l'invention, le métal alcalin et/ou alcalino-terreux fait partie du composé fluoré de l'aluminium.

Selon une première variante du système catalytique selon l'invention, le composé fluoré d'aluminium du support comprend le métal alcalin et/ou alcalino-terreux. Dans cette variante de l'invention, le composé fluoré d'aluminium est avantageusement un fluoroaluminate de métal alcalin et/ou alcalino-terreux. Particulièrement avantageux sont les fluoroaluminates de métaux alcalins. De bons résultats ont été obtenus avec les hexafluoroaluminates de lithium et/ou de sodium.

Selon une deuxième variante du système catalytique selon l'invention, qui est préférée, le support est obtenu par fluoration d'un aluminate d'un métal alcalin et/ou alcalino-terreux. Dans cette variante, l'aluminate comprend de préférence un aluminate de métal alcalin. D'une manière particulièrement préferée, l'aluminate comprend un aluminate de sodium et/ou de lithium. D'une manière tout particulièrement préférée, l'aluminate comprend un aluminate de lithium correspondant à la formule brute LiAl₅O₈. Dans la deuxième variante qui vient d'être décrite, on utilise avantageusement un aluminate de métal alcalin et/ou alcalino-terreux obtenu par la technique décrite dans la demande de brevet EP-A-0486091 [SOLVAY (Société Anonyme)] selon laquelle on met en contact une alumine et une solution d'un composé alcalin et/ou alcalino-terreux susceptible de former un oxyde, on élimine l'eau, puis on soumet le produit obtenu à un cycle thermique de calcination pour transformer le compose alcalin et/ou alcalino-terreux en l'oxyde correspondant et pour provoquer la réaction entre l'oxyde alcalin et/ou alcalino-terreux et l'alumine.

Dans la deuxième variante qui vient d'être décrite, la fluoration de l'aluminate peut être effectuée par n'importe quelle technique connue adéquate. Selon un premier mode de réalisation, la fluoration peut être effectuée par une ou plusieurs imprégnations de l'aluminate à l'aide d'une solution aqueuse contenant du fluorure d'ammonium ou de l'acide fluorhydrique, suivies d'un séchage et d'une calcination de l'aluminate imprégné. Dans ce mode de réalisation, la concentration de la solution aqueuse est de préférence d'au moins 10 % en poids. Dans le cas d'une solution aqueuse de fluorure d'ammonium, la concentration est, de manière particulièrement préférée, d'au moins 20 % en poids. Chaque imprégnation peut être réalisée par n'importe quelle technique connue. On utilise de préférence la technique dite du "volume poreux". Cette technique connue est décrite dans le traité de Charles N. SATTERFIELD cité plus haut. Le séchage de l'aluminate imprégné est habituellement effectué à une température d'au moins 50 °C, de préférence d'au moins 80 °C. Habituellement, la température de séchage ne dépasse pas 500 °C. De préférence, elle ne dépasse pas 250 °C. La durée de séchage est généralement d'au moins 1 heure, de préférence d'au moins 2 heures. En général, la durée de séchage ne dépasse pas 48 heures. De préférence, elle ne dépasse pas 24 heures. La calcination peut être effectuée sous atmosphère d'air ou d'un gaz inerte. La calcination est généralement effectuée à une température d'au moins 250 °C, de préférence d'au moins 500 °C. En général, la température de calcination ne dépasse pas 1500 °C. De préférence, elle ne dépasse pas 1100 °C. La durée de calcination est, en général, d'au moins 1 heure, de préférence d'au moins 2 heures. Habituellement, la durée de calcination ne dépasse pas 24 heures. De préférence, elle ne dépasse pas 10 heures.

Selon un deuxième mode de réalisation de la deuxième variante susdite de l'invention, la fluoration de l'aluminate est effectuée au moyen de fluorure d'hydrogène à l'état gazeux. Dans ce mode de réalisation, qui est préféré, la fluoration de l'aluminate est effectuée en le mettant en présence de fluorure d'hydrogène gazeux, éventuellement mélangé à un gaz inerte, tel que l'azote. La fluoration est de préférence effectuée à une température d'au moins 100 °C, de préférence d'au moins 150 °C. Habituellement, la température de fluoration ne dépasse pas 500 °C. De préférence, elle ne dépasse pas 350 °C. La durée de la fluoration au moyen de fluorure d'hydrogène gazeux est avantageusement d'au moins 5 heures, de préférence d'au moins 10 heures. Habituellement, la durée de la fluoration ne dépasse pas 48 heures. De préférence, elle ne dépasse pas 24 heures.

Dans la deuxième variante du système catalytique selon l'invention, qui vient d'être décrite, la fluoration de l'aluminate de métal alcalin et/ou alcalino-terreux peut être effectuée avant ou après avoir déposé le catalyseur sur le support. Dans le cas où la fluoration est effectuée après avoir déposé le catalyseur sur le support, la fluoration de l'aluminate peut se faire au moyen de fluorure d'hydrogène libéré lors du traitement d'un hydrocarbure chlorofluoré avec de l'hydrogène en présence d'un catalyseur d'hydrogénation. Il a ainsi été observé que lorsqu'on traite un hydrocarbure chlorofluoré au moyen d'hydrogène en présence d'un catalyseur d'hydrogénation déposé sur un aluminate d'un métal alcalin et/ou alcalino-terreux, dans un premier temps du fluorure d'hydrogène est libéré par la réaction, de sorte que l'aluminate est progressivement fluoré in situ pour donner le système catalytique selon l'invention. Une fois ce dernier formé, la réaction de l'hydrocarbure chlorofluoré avec l'hydrogène en présence dudit système catalytique provoque l'hydrodéchloration dudit hydrocarbure chlorofluoré avec formation substantielle concommittante de chlorure d'hydrogène.

Dans la deuxième variante du système catalytique selon l'invention, décrite ci-dessus, on préfère réaliser la fluoration de l'aluminate avant d'y déposer le catalyseur d'hydrogénation.

Dans la deuxième variante susdite, les caractéristiques physiques et chimiques du support dépendent largement des conditions de la fluoration de l'aluminate de métal alcalin et/ou alcalino-terreux. Le produit de la fluoration comprend au moins un composé fluoré de l'aluminium, le métal alcalin et/ou alcalino-terreux et éventuellement de l'aluminate de départ qui n'a pas réagi. Le produit de la fluoration peut notamment comprendre, dans des proportions variables, un composé fluoré de l'aluminium et du métal alcalin et/ou alcalino-terreux et un fluorure d'aluminium.

Selon une troisième variante du système catalytique selon l'invention, le support peut être obtenu en soumettant une alumine à une fluoration et à un traitement avec un sel ou un hydroxyde de métal alcalin et/ou alcalino-terreux. La fluoration peut être effectuée par n'importe quelle technique connue adéquate. Elle est de préférence effectuée au moyen de fluorure d'hydrogène à l'état gazeux, à une température qui ne dépasse pas 500 °C. De manière avantageuse, la température de la fluoration est d'au moins 100 °C. Le traitement avec le sel ou l'hydroxyde de métal alcalin et/ou alcalino-terreux peut avantageusement consister en une imprégnation avec une solution aqueuse ou organique du sel ou de l'hydroxyde, suivie d'un séchage et d'un cycle thermique de calcination. De manière avantageuse, le sel de métal alcalin et/ou alcalino-terreux est choisi parmi les nitrates, les acétates et les formiates. Dans cette troisième variante, le métal alcalin et/ou alcalino-terreux du support est de préférence choisi parmi le magnésium, le césium, le sodium et le lithium. De bons résultats ont été obtenus avec le sodium et/ou le lithium.

Selon un mode de réalisation particulier de la troisième variante susdite de l'invention, la fluoration de l'alumine est effectuée avant le traitement avec le sel ou l'hydroxyde de métal alcalin et/ou alcalino-terreux. Dans ce cas, le traitement avec le sel ou l'hydroxyde de métal alcalin et/ou alcalino-terreux est de préférence effectué avant d'y déposer le catalyseur d'hydrogénation.

Selon un autre mode de réalisation de cette troisième variante de l'invention, le traitement avec le sel ou l'hydroxyde de métal alcalin et/ou alcalino-terreux est effectué avant la fluoration. Dans ce cas, la fluoration de l'alumine traitée avec le sel ou l'hydroxyde de métal alcalin et/ou alcalino-terreux, est de préférence réalisée avant d'y déposer le catalyseur d'hydrogénation pour former le système catalytique.

Selon une quatrième variante du système catalytique selon l'invention qui est spécialement avantageuse, le catalyseur comprend du palladium et au moins un autre métal choisi parmi les éléments du groupe VIII du tableau périodique des éléments. Dans ce cas, le catalyseur comprend de préférence du palladium et du rhodium et/ou du ruthénium. Le rapport atomique du palladium au rhodium et/ou au ruthénium est de préférence d'au moins 0,1. D'une manière particulièrement préférée, ce rapport atomique est d'au moins 0,2. Tout particulièrement préféré est un rapport atomique d'au moins 0,5. De préférence, le rapport atomique du palladium au rhodium et/ou au ruthénium ne dépasse pas 10. D'une manière particulièrement préférée, ce rapport atomique ne dépasse pas 5. Tout particulièrement préféré est un rapport atomique qui ne dépasse pas 3. Dans cette variante de l'invention, le palladium et le rhodium et/ou le ruthénium sont avantageusement introduits dans le système catalytique par imprégnation du support comme exposé plus haut. Dans ce cas, de bons résultats ont été obtenus en introduisant d'abord le palladium, puis le rhodium et/ou le ruthénium dans le système catalytique.

Selon une cinquième variante du système catalytique selon l'invention, le catalyseur est essentiellement constitué de palladium.

Le système catalytique selon l'invention permet d'obtenir une bonne activité et une bonne sélectivité et ne se désactive que lentement lors de sa mise en oeuvre dans des procédés d'hydrogénation. En plus, la régénération du système catalytique est facile et peut être effectuée sous atmosphère d'air.

Le système catalytique selon l'invention peut être utilisé dans tout procédé d'hydrogénation. Il apparaît particulièrement avantageux dans les procédés d'hydrogénation dans lesquels sont formés du fluorure et/ou du chlorure d'hydrogène.

L'invention concerne dès lors également un procédé d'hydrodéchloration d'hydrocarbures chlorés, plus particulièrement d'hydrocarbures chlorofluorés, au moyen d'hydrogène et en présence d'un système catalytique conforme à l'invention.

Par hydrocarbures chlorés, on entend désigner les hydrocarbures comprenant au moins un atome de chlore. Par hydrocarbures chlorofluorés, on entend désigner les hydrocarbures comprenant au moins un atome de chlore et au moins un atome de fluor. L'hydrocarbure chlorofluoré peut être totalement ou partiellement chlorofluoré.

Par définition, l'hydrodéchloration consiste à substituer, au sein d'une molécule chlorée, au moins un atome de chlore par un atome d'hydrogène.

Dans le procédé selon l'invention, l'hydrodéchloration a lieu de préférence en phase gazeuse. Elle s'effectue de préférence à une température d'au moins 100 °C, plus particulièrement d'au moins 200 °C. La température d'hydrodéchloration ne dépasse habituellement pas 500 °C. De préférence, elle ne dépasse pas 400 °C.

La pression à laquelle est effectuée l'hydrodéchloration n'est pas critique en elle-même. Habituellement, on opère sous une pression d'au moins 1 bar. Généralement, la pression ne dépasse pas 50 bar. De préférence, elle ne dépasse pas 10 bar.

L'hydrodéchloration est effectuée au moyen d'hydrogène, éventuellement mélangé à un gaz inerte, tel que l'hélium.

Le rapport molaire hydrogène/hydrocarbure chloré ou chlorofluoré est de préférence d'au moins 0,5, plus particulièrement d'au moins 1. Ce rapport ne dépasse de préférence pas 50. D'une manière particulièrement préférée, il ne dépasse pas 15.

Le temps de contact moyen entre les réactifs et le système catalytique, c'est-à-dire le rapport entre le volume occupé par le système catalytique et le débit total d'alimentation, est de préférence d'au moins 1 seconde, plus particulièrement d'au moins 3 secondes. De préférence, le temps de contact ne dépasse pas 30 secondes. D'une manière particulièrement préférée, le temps de contact ne dépasse pas 15 secondes.

Le procédé d'hydrodéchloration selon l'invention peut être réalisé dans un réacteur à lit fixe ou à lit fluidisé. On utilise de préférence un réacteur comprenant un lit fixe du système catalytique.

Le procédé d'hydrodéchloration selon l'invention permet d'obtenir des taux de transformation élevés et des sélectivités très importantes.

Le procédé selon l'invention présente en outre l'avantage que la désactivation du système catalytique au cours du temps est particulièrement lente et que sa régénération est aisée. La régénération du système catalytique permet de réinstaurer l'activité catalytique de départ. La régénération du système catalytique peut notamment être effectuée simplement à l'aide d'air, d'oxygène ou de vapeur d'eau. Un procédé de régénération ayant donné de bons résultats consiste à balayer le réacteur contenant le système catalytique au moyen d'air pendant quelques heures, par exemple pendant 10 à 20 heures, à une température d'au moins 300 °C, de préférence d'au moins 350 °C. De préférence, la régénération est effectuée à une température ne dépassant pas 400 °C.

Le procédé selon l'invention s'applique notamment au traitement d'hydrodéchloration de chlorofluoroalcanes et de chlorofluoroalcènes. Il trouve une application particulièrement intéressante dans la fabrication du difluorométhane au départ de chlorodifluorométhane.

Dans le cas particulier où le procédé selon l'invention est appliqué à la fabrication de difluorométhane par hydrodéchloration de chlorodifluorométhane, on effectue de préférence l'hydrodéchloration avec l'hydrogène à une température d'au moins 200 °C. Les meilleures résultats sont obtenus à une température d'au moins 280 °C. De préférence, la température ne dépasse pas 400 °C. D'une manière particulièrement préférée, la température ne dépasse pas 360 °C. Le rapport molaire hydrogène/chlorodifluorométhane est de préférence d'au moins 1. Plus particulièrement préféré est un rapport molaire d'au moins 2. De préférence, le rapport molaire hydrogène/chlorodifluorométhane ne dépasse pas 15. D'une manière particulièrement préférée, le rapport molaire ne dépasse pas 10.

Cette forme de réalisation du procédé selon l'invention permet d'obtenir un taux de transformation élevé du chlorodifluorométhane et une sélectivité pour le difluorométhane très élevée, en général supérieure à 70 % molaire. La désactivation du système catalytique est lente et sa régénération est aisée.

L'invention se trouve plus amplement illustrée par les exemples suivants.

### Exemple 1 (conforme à l'invention)

### 1) Préparation du support

### a) Préparation d'aluminate de lithium :

1 kg d'alumine en billes de 2 à 3 mm de diamètre moyen (surface spécifique = 180 m²/g, volume poreux = 0,38 cm³/g) a été séché à 250 °C pendant 24 h sous un courant d'azote. Une solution contenant 164 g de LiOH.H₂O, dissous dans un mélange de 280 ml d'acide acétique glacial et de 100 ml d'eau, a été ajoutée en plusieurs fractions à l'alumine sèche. L'alumine ainsi imprégnée a ensuite été chauffée sous un balayage d'air jusque 600 °C à raison de 5 °C/min. La température a ensuite été maintenue à 600 °C pendant 4 h puis augmentée jusque 1050 °C, toujours à raison de 5 °C/min. La température a été maintenue à 1050 °C pendant 8 h.

Le composé ainsi obtenu présentait une surface spécifique de 25 m²/g et un volume poreux de 0,35 cm³/g. L'analyse par diffraction aux RX a mis en évidence un aluminate de lithium de structure LiAl₅O₈.

### b) Fluoration de l'aluminate de lithium :

100 g de l'aluminate de lithium obtenu en a) ont été introduits dans un réacteur tubulaire en acier inoxydable (40 cm de long, 2,54 cm de diamètre), et chauffés sous un courant d'azote (1 mol/h) jusque 250 °C. Après 1 heure de séchage, un débit supplémentaire de 1 mol de fluorure d'hydrogène gazeux par heure a été envoyé dans le réacteur et le débit d'azote a alors été diminué progressivement jusqu'à 0,2 mol/h. Après 10 h de traitement, la température a été augmentée à 300 °C. Après 14 h, correspondant à la fin du dégagement d'eau, l'alimentation en fluorure d'hydrogène a été coupée. Un débit de 0,5 mole d'azote par heure a été maintenu pendant 2 jours pour éliminer le fluorure d'hydrogène excédentaire. Le réacteur a ensuite été refroidi jusqu'à environ 20 °C et mis sous vide (≃ 1,5 mbar) pendant environ 30 minutes. On a recueilli du réacteur 148 g de granules présentant une surface spécifique de 5 m²/g et un volume poreux de 0,13 cm³/g. L'analyse par diffraction aux RX a mis en évidences les structures de AlF₃ rhomboédrique et de Li₃AlF₆. Ces granules constituent le support du système catalytique. L'acidité de ce support était de 27 micromoles/g.

### 2) Préparation du système catalytique

20 cm³ (soit 24,5 g) du support obtenu en 1), ont été introduits dans une ampoule de 50 cm³. L'ampoule a été chauffée sous vide (0,1 à 6 mbar) à 125 °C pendant 2 h en vue de sécher et de dégazer le support. Après refroidissement, le support a été imprégné une première fois sous vide à température ambiante par 9,15 g d'une solution aqueuse à 15 % en volume d'acide chlorhydrique concentré comprenant 1,09 g de PdCl₂. Le support imprégné a été placé pendant 2 h sous vide et environ 16 h à pression atmosphérique à température ambiante. Le support imprégné a ensuite été séché durant 3 h sous vide à 125 °C.

Une deuxième imprégnation suivant la même technique, mais avec 7,9 g d'une solution aqueuse contenant 0,94 g de PdCl₂, a ensuite été réalisée.

5 cm³ du support imprégné ont été introduits dans un réacteur constitué d'un tube en acier inoxydable (longueur : 520 mm, diamètre intérieur : 7,7 mm) et traités pendant 2 h à 240 °C sous 3 bar au moyen d'un mélange d'hydrogène et d'hélium dans un rapport volumique 10/90 à un débit de 40 cm³/min, dans le but de réduire le palladium à l'état métallique.

Le système catalytique ainsi obtenu comprenait 5 % en poids de Pd par rapport au poids de support mis en oeuvre.

### 3) Hydrodéchloration de chlorodifluorométhane (HCFC-22) :

Le réacteur contenant le système catalytique, tel que décrit ci-dessus, a ensuite été alimenté à raison de 0,0134 mol de HCFC-22 et de 0,0937 mol d'hydrogène par heure, à 320 °C, sous 3 bar. Le temps de séjour a été évalué à 10,4 s.

Après 2 h de fonctionnement, le taux de conversion du HCFC-22 était de 68 % et la sélectivité en difluorométhane (HFC-32), définie comme la fraction du HCFC-22 ayant réagi qui est transformée en HFC-32, était de 81 % molaire. Le sous-produit principal était du méthane (sélectivité de 16 % molaire).

Après 100 h de fonctionnement, le taux de conversion du HCFC-22 était de 64 % et la sélectivité en HFC-32 était de 82 % molaire.

Après 360 h de fonctionnement, le taux de conversion du HCFC-22 était de 58 % et la sélectivité en HFC-32 était de 84 % molaire.

La température a ensuite été augmentée à 340 °C. Le réacteur a alors été alimenté à raison de 0,0402 mol de HCFC-22 et de 0,161 mol d'hydrogène par heure, sous 5 bar. Le temps de séjour a été évalué à 9 s.

Après 455 h de fonctionnement, le taux de conversion du HCFC-22 était de 50 % et la sélectivité en HFC-32 était de 86 % molaire.

Après 520 h de fonctionnement, le taux de conversion du HCFC-22 était de 19 % et la sélectivité en HFC-32 était de 86 % molaire.

### 4) Régénération :

Le réacteur a été refroidi jusqu'à la température ambiante et les débits en HCFC-22 et en hydrogène ont alors été remplacés par un débit horaire de 0,05 mol d'air sous 1 bar. La température a ensuite été augmentée progressivement jusque 340 °C à raison de 1 °C/min. La température a été maintenue à 340 °C pendant 5 h puis portée à 385 °C pendant 2 h.

La composition catalytique ainsi régénérée a ensuite été soumise à une réduction au moyen d'un mélange d'hydrogène et d'hélium, comme décrit au point 2) ci-dessus.

Le réacteur a ensuite été alimenté aux débits horaires de 0,0134 mol de HCFC-22 et de 0,0937 mol d'hydrogène, à 320 °C, sous 3 bar.

Après 2 h de fonctionnement, le taux de conversion du HCFC-22 était de 62 % et la sélectivité en HFC-32 était de 82 % molaire.

Après 160 h de fonctionnement, le taux de conversion du HCFC-22 était encore de 53 % et la sélectivité en HFC-32 était de 82 % molaire.

### Exemple 2 (de comparaison)

### 1) Préparation du support :

1 kg d'alumine en billes de 2 à 3 mm de diamètre moyen (surface spécifique = 180 m²/g, volume poreux = 0,38 cm³/g) a été séché à 250 °C pendant 24 h sous un courant d'azote.

100 g de cette alumine ont été fluorés selon la méthode décrite à l'exemple 1, point 1b). Le support ainsi fluoré était principalement constitué de AlF₃.

### 2) Préparation du système catalytique :

10 cm³ (10,8 g) de support, obtenu comme décrit ci-dessus ont été imprégnés avec 2,4 g d'une solution aqueuse à 15 % en volume d'acide chlorhydrique concentré contenant 0,16 g de Pd sous forme de PdCl₂, suivant une méthode analogue à celle décrite dans l'exemple 1, point 2).

2 cm³ du support imprégné ont été introduits dans un réacteur et traités au moyen d'un mélange d'hydrogène et d'hélium, comme exposé dans l'exemple 1 point 2).

Le système catalytique ainsi obtenu comprenait 1,5 % en poids de Pd par rapport au poids de support mis en oeuvre.

### 3) Hydrodéchloration de chlorodifluorométhane (HCFC-22) :

Le réacteur contenant le système catalytique, tel que décrit ci-dessus, a ensuite été alimenté à raison de 0,0214 mol de HCFC-22 et de 0,0856 mol d'hydrogène par heure, à 320 °C, sous 3 bar. Le temps de séjour a été évalué à 4,1 s.

Après 2 h de fonctionnement à cette température, le taux de conversion du HCFC-22 était de 99 %, mais la sélectivité en HFC-32 était inférieure à 2 % molaire. Les produits formés étaient le trifluorométhane (HFC-23) (sélectivité de 21 % molaire) et le méthane (sélectivité de 79 % molaire).

Une comparaison des résultats des exemples 1 et 2 fait apparaître le progrès apporté par l'invention en ce qui concerne la sélectivité de la réaction d'hydrodéchloration en HFC-32.

### Exemple 3 (de comparaison)

### 1) Préparation du système catalytique :

Le support utilisé était un charbon actif de la firme NORIT (type NORIT®RX3) ayant une surface spécifique de 1540 m²/g et un volume poreux de 0,77 cm³/g.

Le système catalytique a été préparé suivant une méthode analogue à celle décrite dans l'exemple 1 point 2), sauf que 10 cm³ de support (soit 3,5 g), concassé en grains de 1 à 2 mm, ont été imprégnés avec 2,4 g d'une solution aqueuse à 15 % volume d'acide chlorhydrique concentré contenant 0,17 g de Pd sous forme de PdCl₂.

5 cm³ du support imprégné ont été traités au moyen d'un mélange d'hydrogène et d'hélium, comme exposé dans l'exemple 1, point 2).

Le système catalytique ainsi obtenu comprenait 4,9 % en poids de Pd par rapport au poids de support mis en oeuvre.

### 2) Hydrodéchloration du chlorodifluorométhane (HCFC-22) :

Le réacteur contenant le système catalytique, tel que décrit ci-dessus, a ensuite été alimenté à raison de 0,0134 mol de HCFC-22 et de 0,0937 mol d'hydrogène par heure, à 320 °C, sous 3 bar. Le temps de séjour a été évalué à 10,4 s.

Après 2 h de fonctionnement, le taux de conversion du HCFC-22 était de 76 % et la sélectivité en HFC-32 était de 90 % molaire. Le sous-produit principal était du méthane (sélectivité de 7 % molaire).

Après 130 h de fonctionnement, le taux de conversion du HCFC-22 n'était plus que de 35 % et la sélectivité en HFC-32 était de 87 % molaire.

Une comparaison des résultats des exemples 1 et 3 fait apparaître le progrès apporté par l'invention en ce qui concerne la stabilité de l'activité du système catalytique au cours du temps.

### Exemple 4 (conforme à l'invention)

### 1) Préparation du système catalytique :

Le support utilisé a été celui décrit à l'exemple 1, point 1).

Le système catalytique a été préparé suivant un mode opératoire analogue à celui décrit dans l'exemple 1, point 2), sauf que 6 cm³ de support (7,44 g) ont été imprégnés une première fois avec 2,27 g d'une solution aqueuse à 15 % en volume d'acide chlorhydrique concentré contenant 0,18 g de Pd sous forme de PdCl₂ et qu'une deuxième imprégnation a ensuite été effectuée avec 2 g d'une solution aqueuse à 15 % en volume d'acide chlorhydrique concentré contenant 0,175 g de Ru sous forme de RuCl₃.

3 cm³ du support imprégné ainsi obtenu ont été traités au moyen d'un mélange d'hydrogène et d'hélium, de la manière exposée à l'exemple 1, point 2).

Le système catalytique ainsi obtenu comprenait 2,4 % en poids de Pd et 2,35 % en poids de Ru par rapport au poids de support mis en oeuvre.

### 2) Hydrodéchloration de chlorodifluorométhane (HCFC-22) :

Le réacteur contenant le système catalytique, tel que décrit ci-dessus, a été alimenté à raison de 0,008 mol de HCFC-22 et de 0,056 mol d'hydrogène par heure, à 320 °C, sous 3 bar. Le temps de séjour a été évalué à 10,4 s.

Après 2 h de fonctionnement, le taux de conversion du HCFC-22 était de 83 % et la sélectivité en HFC-32 était de 78 % molaire. Le sous-produit principal était du méthane (sélectivité de 19 % molaire).

Après 450 h de fonctionnement, le taux de conversion du HCFC-22 était de 75 % et la sélectivité en HFC-32 était de 79 % molaire.

### 3) Régénération :

Après que le taux de conversion du HCFC-22 ait chuté à 23 %, le système catalytique a été régénéré sous un débit horaire de 0,05 mol d'air, à 350 °C pendant 12 h et sous 3 bar.

Après la régénération, le réacteur a été à nouveau alimenté à raison de 0,008 mol de HCFC-22 et de 0,056 mol d'hydrogène par heure, à 320 °C, sous 3 bar. Le taux de conversion du HCFC-22 était alors de 79 % et la sélectivité en HFC-32 était de 78 % molaire.

### Exemple 5 (conforme à l'invention)

### 1) Préparation du système catalytique :

Le support utilisé a été celui décrit à l'exemple 1, point 1).

Le système catalytique a été préparé suivant un mode opératoire analogue à celui décrit dans l'exemple 1, point 2), sauf que 10 cm³ de support (13,3 g) ont été imprégnés une première fois avec 4,49 g d'une solution aqueuse à 15 % en volume d'acide chlorhydrique concentré contenant 0,33 g de Pd sous forme de PdCl₂ et qu'une deuxième imprégnation a ensuite été effectuée avec 2,93 g d'une solution aqueuse à 15 % en volume d'acide chlorhydrique concentré contenant 0,33 g de Rh sous forme de RhCl₃.

5 cm³ du support imprégné ainsi obtenu ont été traités au moyen d'un mélange d'hydrogène et d'hélium, de la manière exposée à l'exemple 1, point 2).

Le système catalytique ainsi obtenu comprenait 2,5 % en poids de Pd et 2,5 % en poids de Rh par rapport au poids de support mis en oeuvre.

### 2) Hydrodéchloration de chlorodifluorométhane (HCFC-22) :

Le réacteur contenant le système catalytique, tel que décrit ci-dessus, a ensuite été alimenté à raison de 0,0134 mol de HCFC-22 et de 0,0937 mol d'hydrogène par heure, à 320 °C, sous 3 bar. Le temps de séjour a été évalué à 10,4 s.

Après 2 h de fonctionnement, le taux de conversion du HCFC-22 était de 92 % et la sélectivité en HFC-32 était de 79 % molaire. Le sous-produit principal était du méthane (sélectivité de 19 % molaire).

Après 100 h de fonctionnement, le taux de conversion du HCFC-22 était de 91 % et la sélectivité en HFC-32 était de 80 % molaire.

Après 500 h de fonctionnement, le taux de conversion du HCFC-22 était de 80 % et la sélectivité en HFC-32 était de 83 % molaire.

### Exemple 6 (conforme à l'invention)

### 1) Préparation du support :

15,2 g (20 cm³) d'alumine en billes de 2 à 3 mm de diamètre moyen (surface spécifique = 180 m²/g, volume poreux = 0,38 cm³/g) ont été séchés à 250 °C pendant 24 h sous un courant d'azote et ont été imprégnés avec 7 g d'une solution aqueuse contenant 0,7 g de Na sous forme de NaNO₃. L'alumine ainsi imprégnée a ensuite été séchée, calcinée à 1050 °C et traitée par un excès de fluorure d'hydrogène comme décrit à l'exemple 1, point 1b).

### 2) Préparation du système catalytique :

Le système catalytique a été préparé suivant un mode opératoire analogue à celui décrit dans l'exemple 1, point 2), sauf que 6 cm³ de support (7 g) ont été imprégnés avec 4 g d'une solution aqueuse à 15 % en volume d'acide chlorhydrique concentré contenant 0,35 g de Pd sous forme de PdCl₂.

2 cm³ du support imprégné ainsi obtenu ont été traités au moyen d'un mélange d'hydrogène et d'hélium, de la manière exposée à l'exemple 1, point 2).

Le système catalytique ainsi obtenu comprenait 5 % en poids de Pd par rapport au poids de support mis en oeuvre.

### 3) Hydrodéchloration de chlorodifluorométhane (HCFC-22) :

Le réacteur contenant le système catalytique, tel que décrit ci-dessus, a ensuite été alimenté à raison de 0,0214 mol de HCFC-22 et de 0,0856 mol d'hydrogène par heure, à 320 °C, sous 3 bar. Le temps de séjour a été évalué à 4,2 s.

Après 2 h de fonctionnement, le taux de conversion du HCFC-22 était de 42 % et la sélectivité en HFC-32 (difluorométhane) était de 78 % molaire.

## Revendications

1. Système catalytique contenant, d'une part, un catalyseur d'hydrogénation comprenant au moins un métal choisi parmi les éléments du groupe VIII du tableau périodique des éléments et, d'autre part, un support dudit catalyseur, comprenant au moins un composé fluoré d'aluminium, caractérisé en ce que le support comprend au moins un métal alcalin et/ou alcalino-terreux faisant partie du composé fluoré d'aluminium.

2. Système catalytique selon la revendication 1, caractérisé en ce que le support contient l'aluminium et le métal alcalin et/ou alcalino-terreux dans un rapport atomique aluminium/(métal alcalin et/ou alcalino-terreux) de 0,2 à 22.

3. Système catalytique selon la revendication 1 ou 2, caractérisé en ce que le support comprend un fluoroaluminate de métal alcalin et/ou alcalino-terreux.

4. Système catalytique selon la revendication 3, caractérisé en ce que le fluoroaluminate est selectionné parmi les hexafluoroaluminates de lithium et/ou de sodium.

5. Système catalytique selon l'une quelconque des revendications 1 à 4, caractérisé en ce que le support est obtenu par fluoration d'un aluminate de métal alcalin et/ou alcalinoterreux.

6. Système catalytique selon la revendication 5, caractérisé en ce que l'aluminate comprend un aluminate de lithium de formule LiAl₅O₈.

7. Système catalytique selon la revendication 5 ou 6, caractérisé en ce que la fluoration de l'aluminate est effectuée au moyen de fluorure d'hydrogène à l'état gazeux.

8. Système catalytique selon l'une quelconque des revendications 1 à 7, caractérisé en ce que le catalyseur d'hydrogénation comprend du palladium.

9. Système catalytique selon la revendication 8, caractérisé en ce que le catalyseur d'hydrogénation comprend du palladium et au moins un métal choisi parmi le rhodium et le ruthénium, dans un rapport atomique palladium/(rhodium et/ou ruthénium) d'environ 0,1 à 10.

10. Système catalytique selon l'une quelconque des revendications 1 à 9, caractérisé en ce que la quantité du catalyseur d'hydrogénation sur le support est de 0,5 à 15 % en poids par rapport au poids du support.

11. Procédé d'hydrodéchloration d'hydrocarbures chlorés au moyen d'hydrogène et en présence d'un système catalytique conforme à l'une quelconque des revendications 1 à 10.

12. Procédé selon la revendication 11, caractérisé en ce qu'il est appliqué à des hydrocarbures chlorofluorés.

13. Procédé selon la revendication 11 ou 12, caractérisé en ce que l'hydrodéchloration est effectuée à une température de 200 à 400 °C, à une pression de 1 à 50 bar et avec un rapport molaire hydrogène/hydrocarbure chloré ou chlorofluoré de 1 à 15.

14. Procédé selon la revendication 12 ou 13, caractérisé en ce qu'il est appliqué à l'hydrodéchloration de chlorofluoroalcanes ou de chlorofluoroalcènes.

15. Procédé selon l'une quelconque des revendications 12 à 14, caractérisé en ce qu'il est appliqué à la fabrication de difluorométhane à partir de chlorodifluorométhane.

## Claims

1. Catalytic system containing, on the one hand, a hydrogenation catalyst comprising at least one metal chosen from the elements of group VIII of the Periodic Table of the Elements and, on the other hand, a support of the said catalyst comprising at least one fluorine-containing aluminium compound, characterized in that the support comprises at least one alkali metal and/or alkaline-earth metal forming part of the fluorine-containing aluminium compound.

2. Catalytic system according to Claim 1, characterized in that the support contains aluminium and the alkali metal and/or alkaline-earth metal in an aluminium/(alkali metal and/or alkaline-earth metal) atomic ratio of 0.2 to 22.

3. Catalytic system according to Claim 1 or 2, characterized in that the support comprises an alkali metal and/or alkaline-earth metal fluoroaluminate.

4. Catalytic system according to Claim 3, characterized in that the fluoroaluminate is selected from lithium and/or sodium hexafluoroaluminates.

5. Catalytic system according to any one of Claims 1 to 4, characterized in that the support is obtained by fluorination of an alkali metal and/or alkaline-earth metal aluminate.

6. Catalytic system according to Claim 5, characterized in that the aluminate comprises a lithium aluminate of formula LiAl₅O₈.

7. Catalytic system according to Claim 5 or 6, characterized in that fluorination of the aluminate is carried out by means of hydrogen fluoride in the gas state.

8. Catalytic system according to any one of Claims 1 to 7, characterized in that the hydrogenation catalyst comprises palladium.

9. Catalytic system according to Claim 8, characterized in that the hydrogenation catalyst comprises palladium and at least one metal chosen from rhodium and ruthenium in a palladium/(rhodium and/or ruthenium) atomic ratio from approximately 0.1 to 10.

10. Catalytic system according to any one of Claims 1 to 9, characterized in that the amount of the hydrogenation catalyst on the support is from 0.5 to 15% by weight with respect to the weight of the support.

11. Process for the hydrodechlorination of chlorinated hydrocarbons by means of hydrogen and in the presence of a catalytic system in accordance with any one of Claims 1 to 10.

12. Process according to Claim 11, characterized in that it is applied to chlorofluorinated hydrocarbons.

13. Process according to Claim 11 or 12, characterized in that the hydrodechlorination is carried out at a temperature of 200 to 400°C, at a pressure of 1 to 50 bar and with a hydrogen/chlorinated or chlorofluorinated hydrocarbon molar ratio of 1 to 15.

14. Process according to Claim 12 or 13, characterized in that it is applied to the hydrodechlorination of chlorofluoroalkanes or of chlorofluoroalkenes.

15. Process according to any one of Claims 12 to 14, characterized in that it is applied to the manufacture of difluoromethane from chlorodifluoromethane.

## Patentansprüche

1. Katalytisches System, das einerseits einen Hydrierungskatalysator, der wenigstens ein Metall, das unter den Elementen der Gruppe VIII des Periodensystems der Elemente ausgewählt ist, umfasst, und andererseits einen Träger besagten Katalysators, der wenigstens eine fluorierte Aluminiumverbindung umfasst, enthält, dadurch gekennzeichnet, dass der Träger wenigstens ein Alkali- und/oder Erdalkalimetall umfasst, das Teil der fluorierten Aluminiumverbindung ist.

2. Katalytisches System gemäß Anspruch 1, dadurch gekennzeichnet, dass der Träger das Aluminium und das Alkali- und/oder Erdalkalimetall in einem Atomverhältnis Aluminium/(Alkali- und/oder Erdalkalimetall) von 0,2 bis 22 enthält.

3. Katalytisches System gemäß Anspruch 1 oder 2, dadurch gekennzeichnet, dass der Träger ein Alkali- und/oder Erdalkalifluoroaluminat umfasst.

4. Katalytisches System gemäß Anspruch 3 dadurch gekennzeichnet, dass das Fluoroaluminat unter den Lithium- und/oder Natriumhexafluoroaluminaten ausgewählt ist.

5. Katalytisches System gemäß einem der Ansprüche 1 bis 4, dadurch gekennzeichnet, dass der Träger durch Fluorierung eines Alkali- und/oder Erdalkalimetallaluminats erhalten wird.

6. Katalytisches System gemäß Anspruch 5, dadurch gekennzeichnet, dass das Aluminat ein Lithiumaluminat der Formel LiAl₅O₈ umfasst.

7. Katalytisches System gemäß Anspruch 5 oder 6, dadurch gekennzeichnet, dass die Fluorierung des Aluminats mittels Fluorwasserstoff in gasförmigem Zustand ausgeführt wird.

8. Katalytisches System gemäß einem der Ansprüche 1 bis 7, dadurch gekennzeichnet, dass der Hydrierungskatalysator Palladium umfasst.

9. Katalytisches System gemäß Anspruch 8, dadurch gekennzeichnet, dass der Hydrierungskatalysator Palladium und wenigstens ein Metall, das unter Rhodium und Ruthenium ausgewählt ist, in einem Atomverhältnis Palladium/(Rhodium und/oder Ruthenium) von ungefähr 0,1 bis 10 umfasst.

10. Katalytisches System gemäß einem der Ansprüche 1 bis 9, dadurch gekennzeichnet, dass die Menge des Hydrierungskatalysators auf dem Träger 0,5 bis 15 Gew.-%, bezogen auf das Gewicht des Trägers, beträgt.

11. Verfahren zur Hydrodechlorierung von Chlorkohlenwasserstoffen mittels Wasserstoff und in Gegenwart eines katalytischen Systems gemäß einem der Ansprüche 1 bis 10.

12. Verfahren gemäß Anspruch 11, dadurch gekennzeichnet, dass es auf Chlorfluorkohlenwasserstoffe angewendet wird.

13. Verfahren gemäß Anspruch 11 oder 12, dadurch gekennzeichnet, dass die Hydrodechlorierung bei einer Temperatur von 200 bis 400 °C, bei einem Druck von 1 bis 50 bar und mit einem Molverhältnis Wasserstoff/Chlor- oder Chlorfluorkohlenwasserstoff von 1 bis 15 ausgeführt wird.

14. Verfahren gemäß Anspruch 12 oder 13, dadurch gekennzeichnet, dass es bei der Hydrodechlorierung von Chlorfluoralkanen oder Chlorfluoralkenen ange wendet wird.

15. Verfahren gemäß einem der Ansprüche 12 bis 14, dadurch gekennzeichnet, dass es für die Herstellung von Difluormethan aus Chlordifluormethan angewendet wird.
